# EUROPEAN PATENT APPLICATION

(11) **EP 4 478 051 A1**
(43) Date of publication of application: **18.12.2024**
(21) Application number: 23788311.1
(22) Date of filing: 10.04.2023
(51) Int. Cl.: G01N 33/68, C12N 15/16, C12Q 1/37, G01N 27/62

(54) **METHOD FOR QUANTIFYING ACTIVE OREXIN A**

(30) Priority: 11.04.2022 JP 2022065117
(71) Applicant: Eisai R&D Management Co., Ltd., Tokyo 112-8088 (JP)
(72) Inventor: HORIE, Kanta, Tsukuba-shi, Ibaraki 300-2635 (JP); TAHARA, Kazuhiro, Tsukuba-shi, Ibaraki 300-2635 (JP); YUMOTO, Emiko, Tsukuba-shi, Ibaraki 300-2635 (JP)
(74) Representative: Hoffmann Eitle
(86) International application number: PCT/JP2023/014583
(87) International publication number: WO 2023/199893

(57) **Abstract**

A method for quantifying active orexin A in a specimen, comprising: a step of contacting the specimen with a monoclonal antibody that recognizes the C-terminal side of orexin A to separate orexin A species; a step of digesting the separated orexin A species with a protease to obtain a peptide consisting of an amino acid sequence of SEQ ID NO: 1; and a step of performing mass spectrometry on the peptide.

## Description

### Technical Field

The present disclosure relates to a method for quantifying active orexin A.

### Background Art

Orexin is an intracerebral neurotransmitter involved in maintaining wakefulness. There are two isopeptides of orexin, one of which is orexin A. For example, the disease narcolepsy, which induces excessive sleepiness (hypersomnia), is caused by the loss of orexin neurons, and it is known that the concentration of orexin A in the brain (cerebrospinal fluid) of patients with narcolepsy is significantly low.

Hypersomnia is observed not only in narcolepsy but also in various neurodegenerative diseases. For example, patients with Parkinson's disease often have complications of symptoms of excessive daily sleepiness in addition to motor disorders. The concentration of orexin A in cerebrospinal fluid of Parkinson's disease patients was measured using a currently widely used radioimmunoassay (RIA) manufactured by Phoenix Pharmaceuticals, Inc. and the results showed that although there was a trend that the concentration of orexin A decreased, the change was slight and the variation was large (Non-Patent Literature 1).

Recent studies on orexin A in cerebrospinal fluid have revealed that the orexin A species recognized by an anti-orexin A polyclonal antibody used in currently widely used radioimmunoassay (RIA) manufactured by Phoenix Pharmaceuticals, Inc. (or equivalent) are mostly present in cerebrospinal fluid as short-truncated metabolites (Non-Patent Literature 2).

On the other hand, it has been reported that short-truncated orexin A metabolites have no activity related to the maintenance of wakefulness (Non-Patent Literature 3), suggesting that the activity originally associated with the maintenance of wakefulness of orexin A may be limited to an intact form, which has not undergone metabolism, or to an orexin A species whose metabolism (truncation) is limited to that at the C-terminal site even if the orexin A species has been metabolized.

### Citation List

### Non-Patent Literature

[Non-Patent Literature 1] Miriam, W., et al., "Progressive dopamine and hypocretin deficiencies in Parkinson's disease: Is there an impact on sleet and wakefulness?," J Sleep Res. 2012 Dec; 21(6): 710-717
[Non-Patent Literature 2] Sakai, N., Matsumura, M., Lin, L. et al., "HPLC analysis of CSF hypocretin-1 in type 1 and 2 narcolepsy" Scientific Reports 9, 477 (2019)
[Non-Patent Literature 3] Manja, L. et al., "Structure-Activity Studies of Orexin A and Orexin B at the Human Orexin 1 and Orexin 2 Receptors Led to Orexin 2 Receptor Selective and Orexin 1 Receptor Preferring Ligands," J. Med. Chem. 2004, 47, 5, 1153-1160

### Summary of Invention

### Technical Problem

An object of the present disclosure is to provide a method for quantifying active orexin A.

### Solution to Problem

The present inventors have conducted extensive studies in consideration of the above-described circumstances and have found a method for specifically quantifying active orexin A present in cerebrospinal fluid, thus leading to realization of the present specification.

In other words, the present specification relates to the following [1] to [24].
[1] A method for quantifying active orexin A in a specimen, comprising: a step of contacting the specimen with a monoclonal antibody that recognizes the C-terminal side of orexin A to separate orexin A species; a step of digesting the separated orexin A species with a protease to obtain a peptide consisting of an amino acid sequence of SEQ ID NO: 1; and a step of performing mass spectrometry on the peptide.
[2] The method according to [1], further comprising: a step of purifying the peptide through a solid-phase extraction method before performing the mass spectrometry.
[3] The method according to [1] or [2], further comprising: a step of performing high-performance liquid chromatography before performing the mass spectrometry.
[4] The method according to any one of [1] to [3], wherein the separation of the orexin A species is performed through immunoprecipitation.
[5] A method for quantifying active orexin A in a specimen collected from a subject through the method according to any one of [1] to [4], and assisting in diagnosis of orexin A deficiency, a sleep disorder, a neurodegenerative disease, or a traumatic brain injury based on the quantification results.
[6] The method according to [5], which is a method for assisting in the diagnosis of the neurodegenerative disease.
[7] A method for quantifying active orexin A in a specimen collected from a subject through the method according to any one of [1] to [4], and diagnosing orexin A deficiency, a sleep disorder, a neurodegenerative disease, or a traumatic brain injury based on the quantification results.
[8] The method according to [7], which is a method for diagnosing the neurodegenerative disease.
[9] The method according to any one of [5] to [8], wherein the specimen collected from the subject is cerebrospinal fluid.
[10] The method according to any one of [5] to [9], wherein the neurodegenerative disease is a synucleinopathy or tauopathy.
[11] The method according to any one of [5] to [10], wherein the neurodegenerative disease is Parkinson's disease, Alzheimer's disease, or mild cognitive impairment.
[12] The method according to [3], wherein the separation of the orexin A species is performed through immunoprecipitation.
[13] A method for quantifying active orexin A in a specimen collected from a subject through the method according to [12], and assisting in diagnosis of orexin A deficiency, a sleep disorder, a neurodegenerative disease, or a traumatic brain injury based on the quantification results.
[14] The method according to [13], which is a method for assisting in the diagnosis of the neurodegenerative disease.
[15] The method according to [13], wherein the specimen collected from the subject is cerebrospinal fluid.
[16] The method according to [13], wherein the neurodegenerative disease is a synucleinopathy or tauopathy.
[17] The method according to [13], wherein the neurodegenerative disease is Parkinson's disease, Alzheimer's disease, or mild cognitive impairment.
[18] A method for quantifying active orexin A in a specimen collected from a subject through the method according to [12], and diagnosing orexin A deficiency, a sleep disorder, a neurodegenerative disease, or a traumatic brain injury based on the quantification results.
[19] The method according to [18], which is a method for diagnosing the neurodegenerative disease.
[20] The method according to [18], wherein the specimen collected from the subject is cerebrospinal fluid.
[21] The method according to [18], wherein the neurodegenerative disease is a synucleinopathy or tauopathy.
[22] The method according to [18], wherein the neurodegenerative disease is Parkinson's disease, Alzheimer's disease, or mild cognitive impairment.
[23] A method for quantifying active orexin A in a specimen collected from a subject through the method according to any one of [1] to [4], and determining circadian rhythm based on the quantification results.
[24] The method according to [23], wherein the specimen collected from the subject is cerebrospinal fluid.

### Advantageous Effects of Invention

According to the present disclosure, it is possible to provide a method for quantifying active orexin A.

### Brief Description of Drawings

FIG. 1 is a view representing a target peptide for quantification (peptide consisting of an amino acid sequence of SEQ ID NO: 1) and a graph showing the quantification results in Example 1.
FIG. 2 is a view representing a target peptide for quantification (peptide consisting of an amino acid sequence of SEQ ID NO: 2) and a graph showing the quantification results in Comparative Example 1.
FIG. 3 is a view representing a target peptide for quantification (peptide consisting of an amino acid sequence of SEQ ID NO: 1) and a graph showing the quantification results in Comparative Example 2.
FIG. 4 is a view showing quantitative results of active orexin A in cerebrospinal fluid of healthy individuals, Parkinson's disease patients, Alzheimer's disease patients, and mild cognitive impairment patients.
FIG. 5 is a view showing a correlation between the amount of active orexin A and the amount of orexin A metabolite (C-terminal side-truncated orexin A) in cerebrospinal fluid of healthy individuals, Parkinson's disease patients, Alzheimer's disease patients, and mild cognitive impairment patients.
FIG. 6 is a graph showing temporal changes in the amount of active orexin A in cerebrospinal fluid of healthy individuals.
FIG. 7 is a graph showing temporal changes in the amount of orexin A in cerebrospinal fluid of healthy individuals.

### Description of Embodiments

Hereinafter, the method of the present disclosure will be described in detail. In an amino acid sequence of SEQ ID NO: 1 (XPLPDCCR: SEQ ID NO: 1), the N-terminal X indicates a pyroglutamic acid residue. In an amino acid sequence of SEQ ID NO: 2 (LYELLHGAGNHAAGILTX: SEQ ID NO: 2), the C-terminal X indicates an amidated leucine residue. In an amino acid sequence of SEQ ID NO: 3 (XPLPDCCRQKTCSCRLYELLHGAGNHAAGILTX: SEQ ID NO: 3), the N-terminal X indicates a pyroglutamic acid residue, and the C-terminal X indicates an amidated leucine residue. An amino acid sequence of SEQ ID NO: 4 is TCSCR: SEQ ID NO: 4.

Amethod for quantifying active orexin A according to one embodiment of the present disclosure comprises: a step of contacting a specimen with a monoclonal antibody that recognizes the C-terminal side of orexin A to separate orexin A species; a step of digesting the separated orexin A species with a protease to obtain a peptide consisting of an amino acid sequence of SEQ ID NO: 1; and a step of performing mass spectrometry on the peptide.

Orexin A species is a general term for intact orexin A which has not undergone metabolism and an orexin A metabolite.

Active orexin A is a general term for intact orexin A which has not undergone metabolism and an orexin A metabolism which has undergone metabolism (cleavage) only partly at the C-terminus. Orexin A and its metabolite which are recognized by a monoclonal antibody that recognizes the C-terminal side of orexin A and comprises the amino acid sequence of SEQ ID NO: 1 are active orexin A.

In the present disclosure, quantification refers to determining the amount or concentration of a target substance present in a specimen. Quantification may be absolute or relative quantification. Relative quantification may be determination of a relative amount compared to the amount of another analyte.

The specimen may be cerebrospinal fluid, blood, plasma, serum, or saliva, and is preferably cerebrospinal fluid.

The monoclonal antibody with respect to orexin A is an antibody that recognizes the C-terminal side of orexin A. The C-terminal side of orexin A refers to the 14th-33rd amino acid sequence in the amino acid sequence of SEQ ID NO: 3 (the full length of orexin A). Orexin A can also be said to have an epitope for the above-described monoclonal antibody in the amino acid sequence of SEQ ID NO: 2. The above-described monoclonal antibody may be an antibody produced through common hybridoma technology, or may be a commercially available monoclonal antibody. The above-described monoclonal antibody may be a mouse antibody, a rat antibody, a rabbit antibody, a goat antibody, a chimeric antibody, or a humanized antibody.

The amino acid sequence of SEQ ID NO: 1 is a sequence present near the N-terminus of orexin A, and is the 1 st-8th amino acid sequence in the amino acid sequence of SEQ ID NO: 3. The amino acid sequence of SEQ ID NO: 2 is a sequence present near the C-terminus of orexin A, and is the 16th-33rd amino acid sequence in the amino acid sequence of SEQ ID NO: 3. The peptide consisting of the amino acid sequence of SEQ ID NO: 1 is obtained by digesting the separated orexin A species with a protease. As a protease, trypsin can be used.

In the method of the present disclosure, the separation of orexin A species may be performed through a well-known method. Immunoprecipitation is exemplified as the well-known method, but it is not limited to this. Immunoprecipitation may comprise a step of mixing beads to which antibodies are bound with a specimen comprising cerebrospinal fluid, a step of centrifuging the obtained mixture to collect the beads, and a step of eluting target antigens from the collected beads using a reducing agent.

The method of the present disclosure may further comprise a step of purifying the peptide consisting of the amino acid sequence of SEQ ID NO: 1 through a solid-phase extraction method before performing the mass spectrometry. Examples of solid-phase extraction methods include a reverse-phase solid-phase extraction method, a normal-phase solid-phase extraction method, and an ion-exchange solid-phase extraction method. For example, a filler with silica as a carrier or a filler with a polymer such as an ion exchange resin as a carrier is used as a solid phase. As columns used for a solid-phase extraction method, a column (C18 column) having a filler in which an octadecyl group is bound around a silica gel carrier, a column (C8 column) having a filler in which an octyl group is bound around a silica gel carrier, a column having a filler in which a phenethyl group is bound around a silica gel carrier, a column having a filler in which a diol group is bound around a silica gel carrier, a column having a filler in which an aminopropyl group is bound around a silica gel carrier, and the like can be used.

In the method of the present disclosure, liquid chromatography may be performed before performing mass spectrometry. In other words, the mass spectrometry on the present disclosure may be liquid chromatography-mass spectrometry. An example of the analysis conditions for liquid chromatography-mass spectrometry will be shown in examples.

By using the method of the present disclosure, active orexin A in a specimen collected from a subject is quantified, and based on the quantification results, it is possible to diagnose or assist in the diagnosis of orexin A deficiency, a sleep disorder, a neurodegenerative disease, or a traumatic brain injury. In addition, patients with these diseases and the like can be identified through the method of the present disclosure. Examples of sleep disorders include difficulty falling asleep, awakening during the night, shortened sleep duration, and difficulty waking up. Examples of neurodegenerative diseases include synucleinopathies and tauopathies, and more specific examples thereof include Parkinson's disease, Alzheimer's disease, mild cognitive impairment, narcolepsy, dementia with Lewy bodies, frontotemporal lobar degeneration, progressive supranuclear palsy, corticobasal degeneration, Huntington's disease, dystonia, prion disease, chorea-acanthocytosis, and adrenoleukodystrophy. Diagnosing or assisting in the diagnosis of traumatic brain injury includes identifying sleep disorders resulting from traumatic brain injury.

To diagnose or assist in the diagnosis of orexin A deficiency, a sleep disorder, a neurodegenerative disease, or a traumatic brain injury, for example, it is sufficient as long as an approximate straight line showing the correlation between the amount of active orexin A and the amount of orexin A metabolite (C-terminal-truncated) in cerebrospinal fluid of patients already diagnosed with orexin A deficiency, a sleep disorder, a neurodegenerative disease, or a traumatic brain injury is created and compared with an approximate straight line based on quantification results of undiagnosed patients (subjects) therewith. In some embodiments, the method described in the present specification comprises a step of determining whether the amount of active orexin A exceeds a predetermined cutoff value. The above-described predetermined cutoff value may be the amount of active orexin A determined based on receiver operating characteristic (ROC) analysis.

Subjects may or may not have been previously diagnosed with orexin A deficiency, a sleep disorder, a neurodegenerative disease, or a traumatic brain injury before being diagnosed or assisted in diagnosis through the method of the present disclosure. In the latter case, the method of the present disclosure may be performed to examine the progression of the disease, the level of healing, and the like.

### Examples

Hereinafter, the present disclosure will be described in more detail based on examples, but the present disclosure is not limited to the following examples. A normal control (NC) means a healthy individual, PD means a Parkinson's disease patient, AD means an Alzheimer's disease patient, and MCI means a mild cognitive impairment patient. Hereinafter, when simply described as "%," it means "w/v%" (mass/volume%).

### (Reference Example 1)

### <Preparation of immunoprecipitation beads>

(1) A solution comprising 0.2 mg of anti-orexin A mouse monoclonal antibody (recognizing the C-terminal side of orexin A, manufactured by FUJIFILM Wako Pure Chemical Corporation, product number: 287-98321 (formerly manufactured by Wako Pure Chemical Industries, Ltd., former product number: 283-98323)) was placed in an 8 kDa ultrafiltration dialysis membrane-attached plastic tube (GE Healthcare Cat #80648413), and solvent replacement was performed in 2 L of 0.1 M NaHCO3 and 0.5 M NaCl at a pH of 8.3 (hereinafter referred to as coupling buffer) (allowed to stand overnight in an environment of 4°C for approximately 16 hours).
(2) 0.25 g of CNBr-activated Sepharose 4B beads (manufactured by Cytiva) weighed and stirred in 1 L of a 1 mM hydrochloric acid aqueous solution for 20 minutes.
(3) The beads were collected on a glass Buchner funnel and washed with 25 mL of a 1 mM hydrochloric acid aqueous solution.
(4) The beads were washed twice with 25 mL of coupling buffer.
(5) The beads were collected in a 50 mL conical tube and centrifuged at 3,500 rpm for 5 minutes.
(6) All of the supernatant was removed, and the antibody solution after solvent replacement was added thereto.
(7) Coupling buffer was added visually thereto until its volume was equal to the volume of the beads.
(8) The beads were rotated and stirred at room temperature for 2 hours.
(9) The beads were centrifuged at 3,500 rpm for 5 minutes, and the supernatant was removed.
(10) The beads were washed three times with 25 mL of coupling buffer (centrifugation at 3,500 rpm for 5 minutes and removal of the supernatant were repeated).
(11) The beads were centrifuged at 3,500 rpm for 5 minutes, the supernatant was removed, and then a 1 M ethanolamine aqueous solution (pH 8 to 8.5, confirmed with pH test paper) was added thereto until its volume was equal to the volume of the beads, and the beads were centrifuged at room temperature for 2 hours.
(12) The beads were collected on a glass Buchner funnel and washed with 25 mL of 0.1 M sodium acetate + 0.5 M sodium chloride buffer (pH 4.0) that had been pre-cooled.
(13) The beads were further washed with 25 mL of 0.1 M tris-hydrochloric acid + 0.5 M sodium chloride buffer (pH 8.0) that had been pre-cooled.
(14) The washing in the above-described pH 4.0 buffer and pH 8.0 buffer was repeated a total of four times.
(15) The beads were washed twice with 25 mL of phosphate-buffered physiological saline (PBS).
(16) A 50 volume% slurry solution was prepared using 25 mL of PBS and stored at 4°C.

### (Example 1)

### <Quantification of specific orexin A in cerebrospinal fluid (CSF)>

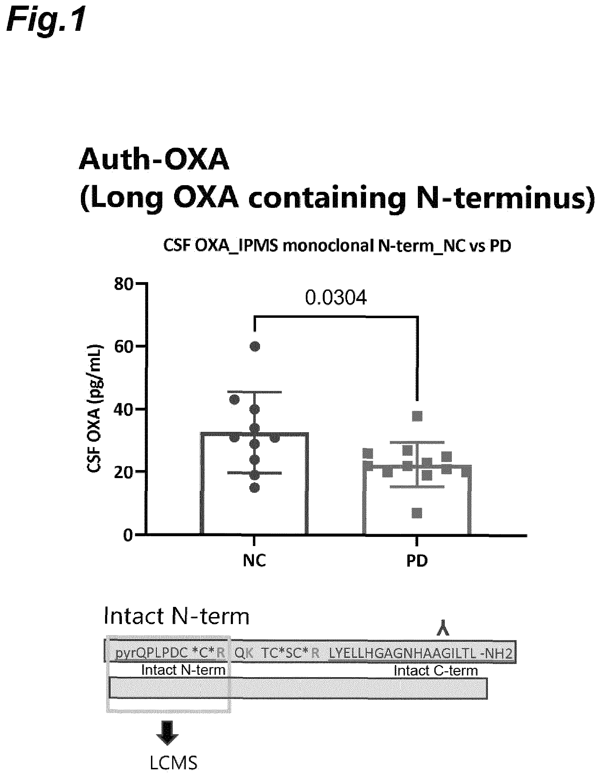

(1) 400 µL of PD-derived CSF (or 500 pg of synthetic orexin Apeptide dissolved in 400 µL of 1% BSA as a standard solution, or 400 µL of 1% BSA as a blank solution) was added to a polypropylene tube.
(2) 500 pg of stable isotope-labeled (leucine 13C-labeled) orexin A peptide (5 µL of a 100 ng/mL solution) was added.
(3) 30 µL of the immunoprecipitation beads (50 volume% slurry solution) obtained in Reference Example 1 were added thereto.
(4) The beads were rotated and stirred at room temperature for 1.5 hours.
(5) Centrifugation was performed at 3,000 rpm for 1 minute, the supernatant was collected in a new polypropylene tube, and then 1 mL of 25 mM triethylammonium bicarbonate (TEABC) was added thereto to wash the beads.
(6) Centrifugation was performed at 3,000 rpm for 1 minute, the supernatant was removed, and then 1 mL of 25 mM TEABC was added thereto to wash the beads. This washing step was repeated again for a total of three times.
(7) Centrifugation was performed at 3,000 rpm for 1 minute, the supernatant was removed, and then 20 µL of 5 mM dithiothreitol (in 25 mM TEABC) was added thereto and incubated at 65°C for 30 minutes.
(8) 20 µL of 10 mM iodoacetamide (in 25 mM TEABC) was added thereto and incubated in a dark place at room temperature for 30 minutes.
(9) 40 µL of 10 µg/mL trypsin (in 25 mM TEABC) was added thereto and incubated at 37°C overnight (for about 16 hours).
(10) After adding 100 µL of 25 mM TEABC thereto and mixing them together, centrifugation was performed at 3,000 rpm for 3 minutes and the supernatant was collected.
(11) TopTip C18 (Glygen) was used to desalinate the supernatant under the following conditions (a) to (e).
   (a) 50 µL of a 60% acetonitrile aqueous solution + 0.1% formic acid was added to a column and centrifuged under the conditions of 3,000 rpm for 3 minutes.
   (b) 200 µL of water + 0.1% formic acid was added to the column and centrifuged under the conditions of 3,000 rpm for 3 minutes.
   (c) The supernatant obtained in the above-described procedure (10) was added to the column and centrifuged under the conditions of 3,000 rpm for 3 minutes.
   (d) 200 µL of water + 0.1% formic acid was added to the column and centrifuged under the conditions of 3,000 rpm for 3 minutes.
   (e) 100 µL of a 60% acetonitrile aqueous solution + 0.1% formic acid was added to the column and centrifuged under the conditions of 3,000 rpm for 3 minutes, and then a sample was collected in a 1.5 mL polypropylene tube.
(12) The collected sample was stored in an environment of -80°C for 15 minutes, and the solution was frozen.
(13) The sample was dried under reduced pressure in a Speed-Vac system.
(14) 27.5 µL of a 2% acetonitrile aqueous solution + 0.1% formic acid was added to the sample after drying under reduced pressure to perform redissolution.
(15) Centrifugation was performed under the conditions of 15,000 rpm for 3 minutes, and 25 µL of the supernatant was transferred to an LC/MS vial.
(16) Active orexin A was quantified through precise quantification of the peptide consisting of the amino acid sequence of SEQ ID NO: 1 under conditions as follows using Orbitrap Fusion Lumos LC/MS system. The results are shown in FIG. 1. As shown in FIG. 1, active orexin A in CSF of PD patients was significantly reduced compared to NCs as a result of a t-test.

### (Conditions for quantitation)

Column: NANO HPLC CAPILLARY COLUMN (NTCC-360/100) manufactured by Nikkyo Technos Co., Ltd., inner diameter 100 µm, 150 mm
Column temperature: Room temperature
Flow rate: 500 nL/min
Injection amount: 5 µL
Solvents:
   Mobile phase A 4% acetonitrile aqueous solution + 0.5% acetic acid
   Mobile phase B 80% acetonitrile aqueous solution + 0.5% acetic acid
   1% B (0 min) - 1% B (5 min) - 37% B (20 min) - 68% B (25 min) - 99% B (26 min) - 99% B (30 min)
Spray voltage: 2000 V
Sweep gas: 1 Arb
Ion transfer tube temperature: 275°C
Parallel reaction mode conditions
   Quadrupole isolation window: 1 m/z
   Activation type: HCD
   RF Lens: 60%
   AGC Target: 500,000

The following peptides were detected in the precise quantitation.
C-terminal trypsin-digested peptide (peptide consisting of amino acid sequence of SEQ ID NO: 2) 621.3476 m/z, high-energy collision dissociation (HCD) collision energy 30%, orbitrap resolution 60000
C-terminal trypsin-digested peptide (stable isotope-labeled) 633.0429 m/z, high energy collision dissociation (HCD) collision energy 30%, orbitrap resolution 30000
N-terminal trypsin-digested peptide (peptide consisting of amino acid sequence of SEQ ID NO: 1) 5514.7180 m/z, high-energy collision dissociation (HCD) collision energy 20%, orbitrap resolution 60000
N-terminal trypsin-digested peptide (stable isotope-labeled) 518.2266 m/z, high energy collision dissociation (HCD) collision energy 20%, orbitrap resolution 30000

### (Comparative Example 1)

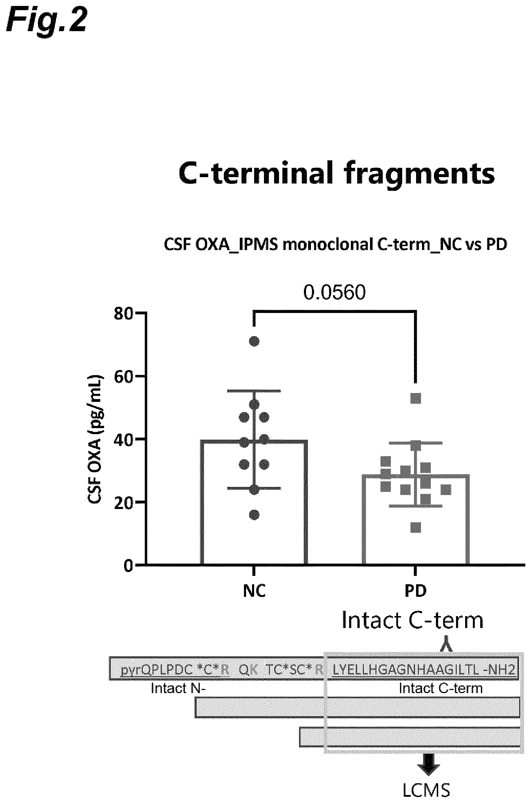

The peptide consisting of the amino acid sequence of SEQ ID NO: 2 was quantified instead of the peptide consisting of the amino acid sequence of SEQ ID NO: 1 in Example 1. The results are shown in FIG. 2. As shown in FIG. 2, the amount of orexin A metabolite with the C-terminus in cerebrospinal fluid was compared between PD patients and NCs, and a t-test performed showed no significant difference.

### (Comparative Example 2)

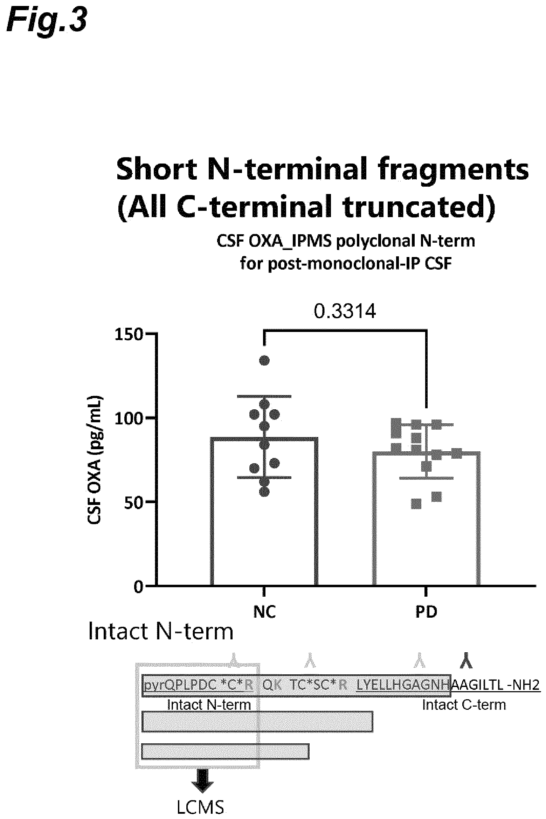

The supernatant collected in the procedure (5) of Example 1 was subjected to immunoprecipitation and mass spectrometry in the same manner as in Example 1 except that immunoprecipitation beads to which polyclonal antibodies (manufactured by FUJIFILM Wako Pure Chemical Corporation, product number: 280-98311) were bound were used instead of immunoprecipitation beads to which monoclonal antibodies were bound, and the orexin A metabolite (C-terminal side-truncated orexin A) was quantified. The results are shown in FIG. 3. As shown in FIG. 3, the amount of C-terminally truncated orexin A metabolite in cerebrospinal fluid was compared between PD patients and NCs, and a t-test performed showed no significant difference.

### (Example 2)

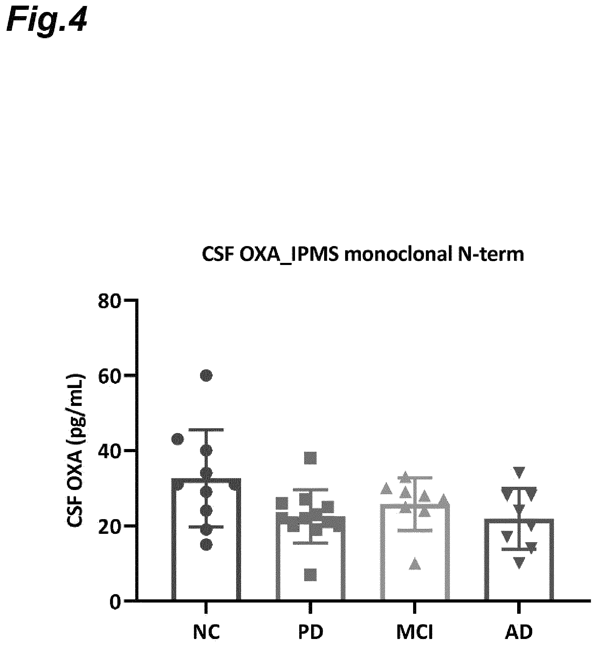

Active orexin A in cerebrospinal fluid from patients exhibiting Alzheimer's disease and mild cognitive impairment was quantified in the same manner as in Example 1. The results are shown in FIG. 4, along with the results of Example 1. There was a trend that the active orexin A in cerebrospinal fluid of patients exhibiting Alzheimer's disease and mild cognitive impairment decreased compared to that of NCs.

### (Reference Example 2)

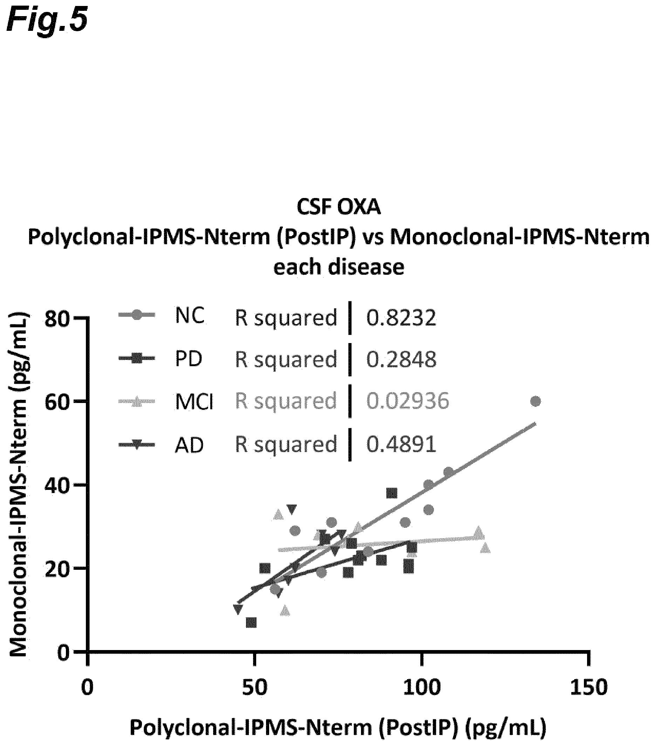

Orexin A metabolite in cerebrospinal fluid from patients exhibiting Alzheimer's disease and mild cognitive impairment was quantified in the same manner as in Example 2. FIG. 5 shows a correlation between the amount of active orexin A measured through the method of Example 1 (vertical axis) and the amount of orexin A metabolite (C-terminal side-truncated orexin A) measured through the method of Comparative Example 2 (horizontal axis) for cerebrospinal fluid from patients exhibiting NC and PD, Alzheimer's disease, and mild cognitive impairment, respectively. As shown in FIG. 5, a high correlation (coefficient of determination = 0.82) was found in NCs, but relatively low correlations (coefficient of determination = 0.029 to 0.49) were found in cerebrospinal fluid from patients exhibiting PD, Alzheimer's disease, and mild cognitive impairment. From this, it is thought that active orexin A in cerebrospinal fluid from patients exhibiting PD, Alzheimer's disease, and mild cognitive impairment is particularly reduced.

### (Example 3)

### <Quantification of active orexin A in cerebrospinal fluid through LC/MS analysis>

<1> 400 µL of each specimen (cerebrospinal fluid collected over time from 5 healthy subjects), a calibration curve solution (a solution obtained by dissolving 0.8 pg, 1.6 pg, 4 pg, 10 pg, 50 pg, 100 pg, or 400 pg of synthetic orexin A peptide was dissolved in 400 µL of 1% BSA), or a blank solution (1% BSA) was added to a polypropylene tube.
<2> Stable isotope (leucine 13C)-labeled orexin A peptide (10 µL of a 200 ng/mL solution) was added thereto.
<3> Immunoprecipitation was performed in the same manner as in (3) to (10) of Example 1, and the supernatant was collected.
<4> The supernatant was desalinated using an OASIS (registered trademark) HLB uElution plate (manufactured by Waters Corporation) under the following conditions <a> to <e>.
<a> 300 µL of a 60% acetonitrile aqueous solution + 0.1% formic acid was added and washed by aspiration.
<b> 500 µL of water + 0.1% formic acid was added to a column and washed by aspiration.
<c> The supernatant obtained in the above-described procedure <3> was added to the column, and aspirated and adsorbed.
<d> 500 µL of water + 0.1% formic acid was added to the column and washed by aspiration.
<e> 100 µL of a 60% acetonitrile aqueous solution + 0.1% formic acid was added to the column and eluted by aspiration, and a sample was collected in a 1.5 mL polypropylene tube.
<5> The collected sample was dried and redissolved in the same manner as in (12) to (14) in Example 1. Active orexin A was quantified through LC/MS in the same manner as in (15) and (16) in Example 1 except that the conditions for quantification were changed to the conditions shown below.

### (Conditions for quantitation)

Column: NANO HPLC CAPILLARY COLUMN (NTCC-360/75) manufactured by Nikkyo Technos Co., Ltd., inner diameter 75 µm, 150 mm
Column temperature: Room temperature
Flow rate: 400 nL/min
Injection amount: 5 µL
Solvents:
   Mobile phase A 4% acetonitrile aqueous solution + 0.5% acetic acid
   Mobile phase B 80% acetonitrile aqueous solution + 0.5% acetic acid
   5% B (0 min) - 5% B (5 min) - 32% B (20 min) - 68% B (25 min) - 99% B (26 min) - 99% B (30 min)
Spray voltage: 2000 V
Sweep gas: 1 Arb
Ion transfer tube temperature: 275°C
Parallel reaction mode conditions
   Quadrupole isolation window: 1 m/z
   Activation type: HCD
   RF Lens: 60%
   AGC Target: 1,000

### (Comparative Example 4)

### <Quantitative analysis of orexin A in human cerebrospinal fluid through EIA measurement>

Quantitative analysis of orexin A in human cerebrospinal fluid was performed using an Orexin A Chemiluminescent EIA kit (manufactured by Phoenix Pharmaceuticals, Inc.) according to the following process. Human orexin A standard peptide (0 pg/mL, 1 pg/mL, 10 pg/mL, 100 pg/mL, 1,000 pg/mL, or 10,000 pg/mL) serially diluted in 1× Assay Buffer, and each specimen for quantitative analysis (each undiluted cerebrospinal fluid collected over time from five healthy subjects) were added to each well. Anti-orexin A polyclonal antibody dissolved in 1 × Assay Buffer was then added thereto, mixed together, and incubated at 4°C overnight. The reaction solution was then aspirated, and biotinylated orexin A was added thereto and incubated at room temperature for 1.5 hours. After washing the resultant four times with 1 × Assay Buffer, streptavidin-HRP diluted by 1,000 times with 1 × Assay Buffer was added thereto and incubated at room temperature for 1 hour. After washing the resultant four times with 1 × Assay Buffer, a substrate solution was added to the wells and incubated at room temperature for 5 to 10 minutes. The relative luminescence was then read in a microplate reader (manufactured by BMG LABTECH). A standard curve was created based on a signal of the human orexin A standard peptide, and the orexin A concentration in cerebrospinal fluid was calculated.

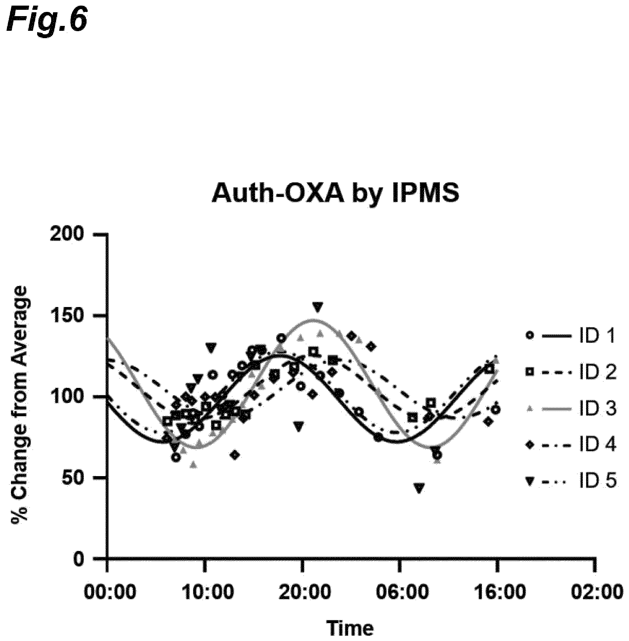

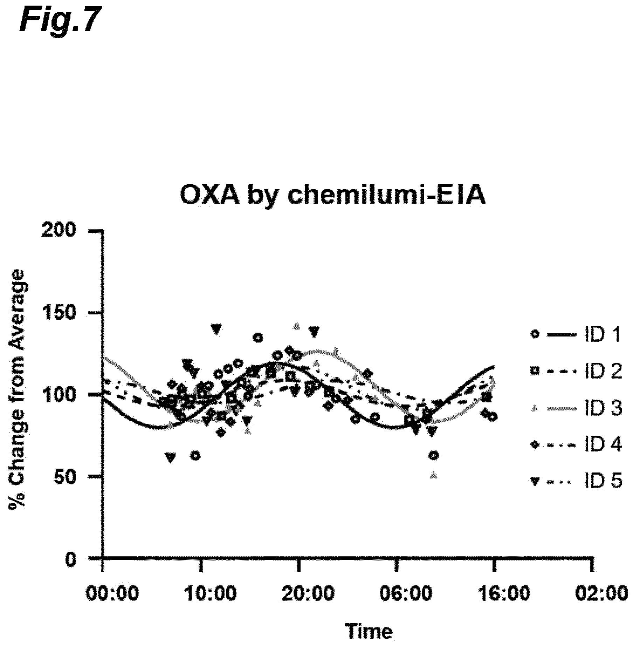

FIG. 6 is a graph showing the amount of active orexin A (vertical axis) measured through the method of Example 3 for the cerebrospinal fluid of five subjects. In FIG. 6, Auth-OXA refers to long orexin A comprising the N-terminus (active orexin A), and IPMS refers to a method for quantifying active orexin A according to the present disclosure. FIG. 7 is a graph showing the amount of orexin A (total amount of N-terminal orexin fragments comprising active orexin A) (vertical axis) measured through the chemiluminescence EIA method in Comparative Example 4. In FIG. 7, OXA refers to orexin A, and chemilumi-EIA refers to the chemiluminescence EIA method. In FIGs. 6 and 7, the values on the vertical axes are relative values (%) when the average of the measurement values for each individual is 100%. In FIGs. 6 and 7, fitting curves calculated with reference to Salomon, RM et al., Biol. Psychiatry 2003, 54, 96-104 are also shown. The horizontal axes in FIGs. 6 and 7 indicate time. ID1 to ID5 in FIGs. 6 and 7 refer to five subjects, respectively.

As shown in FIGs. 6 and 7, a high-amplitude circadian rhythm was observed in the method according to the present disclosure (FIG. 6), while a significantly low amplitude circadian rhythm was observed in the chemiluminescence EIA method in Comparative Example 4 (FIG. 7) (p=0.0025). From this, it was found that the amount of active orexin A varies significantly with time and may be related to the sleep-wake rhythm. In addition, it was also found that the method according to the present disclosure may enable a clearer determination of the subject's circadian rhythm.

### Sequence Listing

International Application FP23-0135 Method for Quantifying Active Orexin A JP23014583 20230410-00070142552300775024_Normal_20230410103949202303231637000140_ P1AP101_FP_0.xml based on International Patent Cooperation Treaty

## Claims

1. A method for quantifying active orexin A in a specimen, comprising:
a step of contacting the specimen with a monoclonal antibody that recognizes the C-terminal side of orexin A to separate orexin A species;
a step of digesting the separated orexin A species with a protease to obtain a peptide consisting of an amino acid sequence of SEQ ID NO: 1; and
a step of performing mass spectrometry on the peptide.

2. The method according to claim 1, further comprising:
a step of purifying the peptide through a solid-phase extraction method before performing the mass spectrometry.

3. The method according to claim 1 or 2, further comprising:
a step of performing high-performance liquid chromatography before performing the mass spectrometry.

4. The method according to any one of claims 1 to 3,
wherein the separation of the orexin A species is performed through immunoprecipitation.

5. A method for quantifying active orexin A in a specimen collected from a subject through the method according to any one of claims 1 to 4, and assisting in diagnosis of orexin A deficiency, a sleep disorder, a neurodegenerative disease, or a traumatic brain injury based on the quantification results.

6. The method according to claim 5, which is a method for assisting in the diagnosis of the neurodegenerative disease.

7. The method according to claim 5 or 6,
wherein the specimen collected from the subject is cerebrospinal fluid.

8. The method according to any one of claims 5 to 7,
wherein the neurodegenerative disease is a synucleinopathy or tauopathy.

9. The method according to any one of claims 5 to 8,
wherein the neurodegenerative disease is Parkinson's disease, Alzheimer's disease, or mild cognitive impairment.
